# EUROPEAN PATENT APPLICATION

(11) **EP 1 557 472 A1**
(43) Date of publication of application: **27.07.2005**
(21) Application number: 03770021.8
(22) Date of filing: 30.10.2003
(51) Int. Cl.: C12P 19/26, C08B 37/08, A61K 31/737, A61K 47/36, A61K 7/00

(54) **SODIUM CHONDROITIN SULFATE, CHONDROITIN SULFATE-CONTAINING MATERIAL AND PROCESSES FOR PRODUCING THE SAME**

(30) Priority: 01.11.2002 JP 2002319418
(71) Applicant: Nippon Barrier Free Co. Ltd., Tokyo 101-0051 (JP)
(72) Inventor: ETO, Tadashi, Tokyo 146-0085 (JP)
(74) Representative: von Samson-Himmelstjerna, Friedrich R.
(86) International application number: PCT/JP2003/013918
(87) International publication number: WO 2004/039994

(57) **Abstract**

After a solution obtained by extracting protein from cartilages of fishes which are enzyme treated is filtered, alcohol is added to a filtrate to deposit sodium chondroitin sulfate. The sodium chondroitin sulfate manufactured thus can be used for a raw material of medicines, medicine derivatives, medicine additives, cosmetics, and food additives. After a solution obtained by extracting protein which is obtained by enzyme treatment of cartilages of fishes is filtered, the filtrate is dried to manufacture a chondroitin-sulfuric-acid-containing substance. The chondroitin-sulfuric-acid-containing substance manufactured thus is used for, for instance, cosmetics, and food materials.

## Description

### Technical Field

The present invention relates to sodium chondroitin sulfates, chondroitin-sulfuric-acid-containing substances and methods of manufacturing thereof.

### Background Art

It has been known from previous studies and researches that there are chondroitin sulfates in cartilages of fishes such as a nose cartilage of salmon. In recent years, utilization of sodium chondroitin sulfate originated from a nose cartilage of salmon for cosmetics or foods has been studied.

Conventionally, as a method of manufacturing sodium chondroitin sulfate from a nose cartilage of salmon or the like, Japanese Patent Application Laid-open No. 2001-231497 and Japanese Patent Application Laid-open No. 2001-247602 have been disclosed. By these conventional methods, after pulverizing and defatting a nose cartilage of salmon or the like at a low temperature from minus 30°C to minus 60°C, it is enzyme-treated by alkaline and heat treatment. The digestive fluid thereof is precipitated with ethanol, filtered, separated with a centrifuge, and dried. Then sodium chondroitin sulfate is obtained by freeze drying after dissolution and filtration with ion-exchange resin.

### [Patent Document 1]

Japanese Patent Application Laid-open No. 2001-231497

### [Patent Document 2]

Japanese Patent Application Laid-open No. 2001-247602

However, since the conventional manufacturing method involves continuous multistage ultrafiltration or filtration after dissolution with deionized water, it is considerably expensive in terms of capital investment, which causes production costs high resulting in extremely expensive sodium chondroitin sulfate. In addition, ethanol precipitation, filtration, and centrifugation have to be repeated several times, which makes the production time longer.

### Disclosure of the Invention

Accordingly, it is an object of the present invention to produce sodium chondroitin sulfate or a chondroitin-sulfuric-acid-containing substance at a cost as low, and in a time as short, as possible.

According to the present invention, a method of manufacturing sodium chondroitin sulfate is provided which comprises the steps of: filtering an extracted solution of protein obtained by enzyme treatment of cartilages of fishes; and precipitating sodium chondroitin sulfate by adding alcohol to the filtrate.

Furthermore, according to the present invention, a method of manufacturing a chondroitin-sulfuric-acid-containing substance is provided which comprises the steps of: filtering an extracted solution of protein obtained by enzyme treatment of cartilages of fishes; and drying the filtrate.

The sodium chondroitin sulfate manufactured thus is used as a raw material of medicines, quasi medicine, medicine additives, cosmetics, or food additives. A chondroitin-sulfuric-acid-containing substance manufactured thus can be used as a raw material for cosmetics or foods, for instance.

### Best Mode for Carrying out the Invention

Preferred embodiments of the present invention will be explained below. First, a method of manufacturing sodium chondroitin sulfate will be explained. A nose cartilage of a salmon is cleaned in warm water of 40°C to 50°C for one hour to two hours. Here, hydrochloric acid may be used for the cleaning. Note that bone and fish meat can be attached to the cartilage.

The same amount of water as that of a nose cartilage and a proteolytic enzyme (for instance, aroase) having solid concentration of 0.1 to 1.0wt% with respect to the nose cartilage are put to the nose cartilage and the mixture is stirred for 3 to 4 hours at 50°C to 60°C. By performing enzyme treatment (extraction) using a proteolytic enzyme, protein is extracted to obtain an extracted solution in which protein is dissolved. It should be noted that when aroase, protease, or the like is used as a proteolytic enzyme, protein can be made to be of comparatively low molecular-weight so that the extracted solution obtained by enzyme treatment contains protein having a comparatively low molecular weight.

Thereafter, the extracted solution is heated at 90°C to 95°C for 5 to 10 minutes to deactivate enzyme. Activated charcoal in an amount of 0.3wt% to 1.0wt% with respect to the extracted solution is further added and stirred for one to two hours at 50°C to 60°C to be defatted, deodorized and decolorized

Then, alkali (for instance, sodium hydroxide) is added to the extracted solution to adjust the pH between 5 to 6.

Thereafter, a filter aid is added and the extracted solution is filtered with a filter press (defatting). The extracted solution after filtration (filtrate) contains protein having comparatively low molecular weight.

Alcohol (for instance, ethanol) with a concentration of 50% or more is added to the extracted solution after filtered to deposit and precipitate sodium chondroitin sulfate. The precipitated crystal is recovered after cooling.

Then, the recovered crystal is dissolved in an amount of water weighing twice as much as the crystal. The extracted solution obtained by dissolving the crystal in water is purified through positive ion exchange resin to remove protein from the aqueous solution. After condensing the extracted solution, it is spray dried. Since sodium chondroitin sulfate is obtained in this way by spray drying the extracted solution, mass, low cost production can be realized.

It should be noted that production of sodium chondroitin sulfate with a higher quality (a medicine grade) can be realized by repetition of removing protein while raising concentration of alcohol to be added to the extracted solution after filtration, and by filtration of the extracted solution again after filtration with a membrane filter or the like.

Another embodiment of the present invention relating to a method of manufacturing sodium chondroitin sulfate will be explained next. First, a nose cartilage is cleaned in warm water at 40°C to 50°C for one to two hours. Here, hydrochchloric acid may be used for the cleaning. Note that bone and fish meat can be attached to the cartilage.

Next, alkali (for instance, sodium hydroxide) with a concentration of 25% is added to the cartilage in an amount of 0.1wt% to 2.0wt% with respect to the cartilage together with a proteolytic enzyme (for instance, pancreatin having 0.2% in solid concentration) and this aqueous solution is stirred at 50°C to 60°C for three hours to four hours. By performing enzyme treatment (extraction) using a proteolytic enzyme, sodium chondroitin sulfate is cut off from protein to obtain an extracted solution in which sodium chondroitin sulfate and protein are extracted and dissolved. Note that salt is combined with pancreatin. When kiwi enzyme (actinidine) or the like is used as a proteolytic enzyme, protein is not comparatively lowered in molecular weight, and can be separated into sodium chondroitin sulfate and other substances. In the extracted solution obtained by using pancreatin or the like as a proteolytic enzyme, protein is contained at a comparatively high molecular weight.

Thereafter, it is heated at the temperature of 90°C to 95°C for 5 to 10 minutes to deactivate enzyme.

Activated charcoal is added in an amount of 0.3wt% to 2.0wt% with respect to the extracted solution, which is stirred at 50°C to 60°C for one hour to two hours to be defatted, deodorized and decolorized.

Thereafter, acetic acid is added to alkaline extracted solution to adjust its pH to 5 to 6.

Then, a filter aid is added to the extracted solution, which is filtered with a filter press.

Alcohol (for instance, ethanol) with a concentration of 50% or more is added to the extracted solution (filtrate) after filtered to deposit and precipitate sodium chondroitin sulfate. The precipitated crystal is recovered after cooling.

Thereafter, the precipitated crystal is stirred again and dehydrated with highly concentrated alcohol and after precipitation by cooling, crystal is recovered by crystal recovery or by centrifugal separation.

As described above, since protein is extracted at a comparatively high molecular weight when pancreatin or the like is used as a proteolytic enzyme, the extract does not require purification through positive ion exchange resin and can be dried under reduced pressure. Thus, expensive investments such as ion exchange resin or a large-size spray dryer are not necessary, and it is possible to manufacture sodium chondroitin sulfate at low costs.

The sodium chondroitin sulfate manufactured thus can be used especially as (for) medicines, quasi medicine, medicine additives, cosmetics, and food additives.

An embodiment of the present invention relating to a method of manufacturing chondroitin-sulfuric-acid-containing substances will be explained next. A salmon cartilage is cleaned with warm water of 40°C to 50°C for one hour to two hours. A proteolytic enzyme (for instance, aroase having solid content of 0.15 to 1.0%) is put in an aqueous solution obtained by adding the same amount of water as the cartilage, which is stirred at 50°C to 60°C for three hours to four hours to perform enzyme treatment to obtain an extracted solution in which protein is extracted

Thereafter, it is heated at 90°C to 95°C for five minutes to 10 minutes to be enzyme deactivated.

Then, activated charcoal is added in an amount of 0.3wt% to 1.0wt% with respect to the extracted solution and is stirred at 50°C to 60°C for one hour to two hours to be defatted, deodorized and decolorized .

Thereafter, alkali (for instance, sodium hydroxide) is added to adjust pH to 5 to 6, and a filter aid is added to the pH adjusted solution, which is then filtered with a filter press.

The filtered extracted solution is dried by spray drying to obtain a chondroitin-sulfuric-acid-containing substance.

In this method of manufacturing the chondroitin-sulfuric-acid-containing substance, alkali (for instance, sodium hydroxide) with a concentration of 25% in an amount of 1 to 5wt% with respect to the amount of cartilage may be added at the time of enzyme treatment.

After filtration, alcohol is added to the filtrate to deposit a chondroitin-sulfuric-acid-containing substance, and the deposited chondroitin-sulfuric-acid-containing substance may be dried.

According to this method of manufacture, chondroitin sulfuric acid, collagen, and the like can be extracted simultaneously as a composite. As for compositions, chondroitin sulfuric acid, collagen, amino acid, hyaluronic acid, and glucosamine can be cited. By selecting the kinds of raw materials, types of enzyme, and methods of enzyme treatment respectively, composition of the chondroitin containing substance can be adjusted to be 5% to 60% of chondroitin sulfuric acid and 5% to 40% of collagen. The chondroitin-sulfuric-acid-containing substance manufactured thus can be used especially as a raw material in the production of cosmetics, food additives, and other food materials.

By selecting a proteolytic enzyme to extract protein, composition of an inclusion can be varied.

As a salmon cartilage used for a raw material, for instance, a portion containing cartilage (half cut) cut from the lower jaw or from behind the eyes of a salmon can be used. Note that it is also possible to use salmon cartilage or the head of a salmon as it is. As raw materials, a head portion, a half-cut, and salmon cartilage are listed in order of increasing processing time and expense, but also chondroitin sulfuric acid contents. Therefore, it is possible to manufacture a variety of products ranging from a low price product having a lower chondroitin sulfuric acid content, to an expensive one having a higher content, based on the raw materials selected.

### [EXAMPLE]

### (EXAMPLE 1)

First, a salmon cartilage is cleaned with warm water of 45°C for two hours to be defatted and deodorized. Then, an aqueous solution is obtained by adding water in the same amount as that of the cartilage. A proteolytic enzyme (aroase) having solid content of 0.1 % is put in the solution and stirred at 55°C for 3 hours to be enzyme treated to obtain an extracted solution. Thereafter, it is heated at 95°C for 5 minutes to be enzyme deactivated. Activated charcoal is added in an amount of 0.3wt% with respect to the extracted solution and stirred at 55°C for one hour to be defatted, deodorized and decolorized. Then, after adjusting pH to 6 with sodium hydroxide, a filter aid is added and the extracted solution is filtered (defatting) with a filter press. The extracted solution obtained thus is precipitated in a solution of 60% ethanol and crystals are recovered after cooling. These crystals are dissolved in twice the volume of water to obtain an extracted solution, which is purified by passing through positive ion exchange resin. Then, it is condensed to 30% and spray dried to obtain sodium chondroitin sulfate.

### (EXAMPLE 2)

First, a salmon cartilage is cleaned with warm water of 45°C for two hours to be defatted and deodorized. An aqueous solution is obtained by adding alkali (sodium hydroxide) with a concentration of 25% in an amount of 2wt% with respect to the amount of cartilage, and a proteolytic enzyme having solid content of 0.2%. It is stirred at 55°C for three hours to perform enzyme treatment to obtain an extracted solution. Thereafter, it is heated at 95°C for 5 minutes to be enzyme deactivated. Activated charcoal is added in an amount of 0.3wt% with respect to the extracted solution, which is stirred at 55°C for one hour to be defatted, deodorized and decolorized. After adjusting pH to 6 by adding acetic acid, a filter aid is added to the solution, which is filtered (defatting) with a filter press. The extracted solution obtained thus is precipitated in a solution of 60% ethanol and crystals are recovered after cooling. The crystal is stirred again in highly concentrated alcohol (90%) and is recovered thereafter by centrifugal separation, which is then dried under reduced pressure to obtain sodium chondroitin sulfate.

### (EXAMPLE 3)

First, a salmon cartilage is cleaned with warm water (45°C) for two hours to be defatted and deodorized. Then, an aqueous solution is obtained by adding the same amount of water as that of the cartilage. A proteolytic enzyme (aroase) having solid content of 0.1 % to 1.0% is put in the solution and stirred at 55°C for 3 hours to be enzyme treated to obtain an extracted solution. Thereafter, it is heated at 95°C for 5 minutes to deactivate enzymes. Activated charcoal is added in an amount of 0.3wt% with respect to the extracted solution and stirred at 55°C for one hour to be defatted, deodorized and decolorized. Then, after adjusting pH to 6 with sodium hydroxide, a filter aid is added and the extracted solution is filtered (defatting) with a filter press. It is dried by spray drying to obtain a chondroitin-sulfuric-acid-containing substance. This chondroitin-sulfuric-acid-containing substance contains 40% of chondroitin sulfuric acid and 20% of collagen, and also contains amino acid, hyaluronic acid, glucosamine, and the like.

### (EXAMPLE 4)

A salmon cartilage is cleaned with warm water of 45°C for two hours to be defatted and deodorized. Water in a half weight of the cartilage is added to obtain a water solution. 0.1% proteolytic enzyme (pancreatin) is added and stirred at 50°C for two hours to perform enzyme treatment to obtain an extracted solution. Thereafter, it is heated at 95°C or higher for 5 minutes to deactivate enzyme. After adjusting pH to 6 by adding acetic acid, a filter aid is added to the solution, which is filtered (defatting) with a filter press. Alcohol with a concentration of 50% is added to the extracted solution while stirring to recover precipitate. The precipitate recovered thus is dried under reduced pressure and a chondroitin-sulfuric-acid-containing substance is obtained. This chondroitin-sulfuric-acid-containing substance contains 60% of chondroitin sulfuric acid and 10% of collagen, and also contains amino acid, hyaluronic acid, glucosamine, and the like.

### (EXAMPLE 5)

First, a salmon cartilage is cleaned with warm water of 45°C for two hours to be defatted and deodorized. An aqueous solution is obtained by adding alkali (sodium hydroxide) with a concentration of 25% in an amount of 2wt% with respect to the amount of cartilage. It is stirred at 55°C for four hours to perform enzyme treatment to obtain an extracted solution. Then, pH of the solution is adjusted to 6 with acetic acid and activated charcoal is added in an amount of 0.3wt% with respect to the extracted solution, which is stirred at 55°C for one hour to deactivate enzyme and to be defatted and decolorized. Then, a filter aid is added, which is filtered (defatting) with a filter press. Thereafter, it is dried by spray drying to obtain a chondroitin-sulfuric-acid-containing substance. This chondroitin-sulfuric-acid-containing substance contains 28% of chondroitin sulfuric acid and 28% of collagen, and also contains amino acid, hyaluronic acid, glucosamine, and the like.

### (EXAMPLE 6)

First, a salmon cartilage is cleaned with warm water (45°C) for two hours to be defatted and deodorized. Then, an aqueous solution is obtained by adding the same amount of water as that of the cartilage. A proteolytic enzyme (papain enzyme) having solid content of 0.25% is put in the solution and stirred at 55°C for 3 hours to be enzyme treated to obtain an extracted solution. Thereafter, it is heated at 95°C for 5 minutes to deactivate enzyme. Activated charcoal is added in an amount of 0.3wt% with respect to the extracted solution and stirred at 55°C for one hour to be defatted, deodorized and decolorized. Then, after adjusting pH to 6 with sodium hydroxide, a filter aid is added and the extracted solution is filtered (defatting) with a filter press. It is further dried by spray drying to obtain a chondroitin-sulfuric-acid-containing substance. This chondroitin-sulfuric-acid-containing substance contains 20% of chondroitin sulfuric acid and 20% of collagen, and also contains amino acid, hyaluronic acid, glucosamine, and the like.

### (EXAMPLE 7)

First, a salmon cartilage is cleaned with warm water (45°C) for two hours to be defatted and deodorized. Then, an aqueous solution is obtained by adding the same amount of water as that of the cartilage. A proteolytic enzyme (kiwi enzyme) having solid content of 0.15% is put in the solution and stirred at 55°C for 3 hours to be enzyme treated to obtain an extracted solution. Thereafter, it is heated at 95°C for 5 minutes to deactivate enzyme. Activated charcoal is added in an amount of 0.3wt% with respect to the extracted solution and stirred at 55°C for one hour to be defatted, deodorized and decolorized. Then, after adjusting pH to 6 with sodium hydroxide, a filter aid is added and the extracted solution is filtered (defatting) with a filter press. It is dried by spray drying to obtain a chondroitin-sulfuric-acid-containing substance. This chondroitin-sulfuric-acid-containing substance contains 40% of chondroitin sulfuric acid and 15% of collagen, and also contains amino acid, hyaluronic acid, glucosamine, and the like.

### (EXAMPLE 8)

First, blood is removed from half cut fish containing a salmon cartilage, and the salmon cartilage is cleaned with warm water (45°C) for two hours to be defatted and deodorized. Then, an aqueous solution is obtained by adding the same amount of water as that of the cartilage. A proteolytic enzyme (aroase) having solid content of 0.1 % is put in the solution and stirred at 55°C for 3 hours to be enzyme treated to obtain an extracted solution. Thereafter, it is heated at 95°C for 5 minutes to deactivate enzyme. Activated charcoal is added in an amount of 0.3wt% with respect to the extracted solution and stirred at 55°C for one hour to be defatted, deodorized and decolorized. Then, after adjusting pH to 6 with sodium hydroxide, a filter aid is added and the extracted solution is filtered (defatting) with a filter press. It is dried by spray drying to obtain a chondroitin-sulfuric-acid-containing substance. This chondroitin-sulfuric-acid-containing substance contains 28% of chondroitin sulfuric acid and 28% of collagen, and also contains amino acid, hyaluronic acid, glucosamine, and the like.

### Industrial Availability

According to the present invention, it is possible to manufacture sodium chondroitin sulfate or a chondroitin-sulfuric-acid-containing substance at low costs in a short time.

## Claims

1. A method of manufacturing sodium chondroitin sulfate, comprising the steps of:
filtering an extracted solution of protein obtained by enzyme treatment of cartilages of fishes; and
precipitating sodium chondroitin sulfate by adding alcohol to said filtrate.

2. The method of manufacturing sodium chondroitin sulfate according to claim 1, wherein alkali is added at the time of said enzyme treatment.

3. The method of manufacturing sodium chondroitin sulfate according to claims 1, further comprising the step of:
purifying sodium chondroitin sulfate deposited by adding alcohol.

4. Sodium chondroitin sulfate being manufactured according to the method of claim 1.

5. The sodium chondroitin sulfate according to claim 4, wherein said sodium chondroitin sulfate is used as a raw material of medicines, quasi medicines, medicine additives, cosmetics, or food additives.

6. A method of manufacturing a chondroitin-sulfuric-acid-containing substance, comprising the steps of:
filtering an extracted solution of protein obtained by enzyme treatment of cartilages of fishes; and
drying said filtrate.

7. A method of manufacturing a chondroitin-sulfuric-acid-containing substance, comprising the steps of:
filtering an extracted solution of protein obtained by enzyme treatment of cartilages of fishes;
precipitating a chondroitin-sulfuric-acid-containing substance by adding alcohol to said filtrate; and
drying said precipitated chondroitin-sulfuric-acid-containing substance.

8. The method of manufacturing a chondroitin-sulfuric-acid-containing substance according to claim 6 or 7, wherein alkali is added at the time of said enzyme treatment.

9. The chondroitin-sulfuric-acid-containing substance manufactured according to the method of claims 6, or 7.

10. The chondroitin-sulfuric-acid-containing substance according to claim 9, wherein said substance is a raw material for either cosmetics or foods.
